# FASCICULE DE BREVET EUROPEEN

(11) **EP 1 330 277 B1**
(45) Date de publication et mention de la délivrance du brevet: **05.01.2005**
(21) Numéro de dépôt: 01978574.0
(22) Date de dépôt: 19.10.2001
(51) Int. Cl.: A61M 5/30

(54) **SERINGUE SANS AIGUILLE SECURISEE A ARCHITECTURE COMPACTE**
NADELLOSE SPRITZE MIT KOMPAKTER BAUWEISE
COMPACT ARCHITECTURE NEEDLELESS SYRINGE

(30) Priorité: 23.10.2000 FR 0013544
(43) Date de publication de la demande: 30.07.2003
(73) Titulaire: Crossject, 75004 Paris Cédex 04 (FR)
(72) Inventeur: ALEXANDRE, Patrick, F-70100 Gray (FR); BROUQUIERES, Bernard, F-83100 Toulon (FR); GAUTIER, Philippe, F-91220 Le Plessis Pate (FR); PIEL, Marc, F-75017 Paris (FR)
(74) Mandataire: Waligorski, Carol
(86) Numéro de dépôt international: PCT/FR2001/003237
(87) Numéro de publication internationale: WO 2002/034317

(56) Documents cités:
- EP-A- 0 853 952
- WO-A-00/44421
- US-A- 5 520 639

## Description

Le domaine technique de l'invention est celui des seringues sans aiguille préremplies et jetables, fonctionnant avec un générateur de gaz, et utilisées pour les injections intradermiques, sous-cutanées et intramusculaires, de principe actif liquide à usage thérapeutique en médecine humaine ou vétérinaire.
Plus particulièrement l'invention concerne une seringue sans aiguille comprenant un corps, un générateur de gaz, une chambre d'expansion desdits gaz, un réservoir contenant le principe actif et un système d'injection présentant au moins un canal d'injection.

Pour les dispositifs d'injection selon l'invention, un principe actif liquide est constitué par un liquide plus ou moins visqueux, ou un mélange de liquide, ou un gel. Le principe actif peut être un solide mis en solution dans un solvant approprié pour l'injection. Il peut également être représenté par un solide pulvérulent mis en suspension plus ou moins concentrée dans un liquide approprié. La granulométrie du principe doit être compatible avec le diamètre des conduits pour éviter les bouchages.

Pour toute la suite du texte les expressions « forme compacte », « architecture compacte » et « géométrie compacte » sont des expressions équivalentes. Elles sont attribuées à des seringues et signifient que leur hauteur a une dimension du même ordre de grandeur que celle de leur longueur tout en préservant un encombrement minimum. De façon plus quantitative, la notion de « même ordre de grandeur » correspond à un rapport « hauteur sur longueur » compris entre 0,5 et 2,5. Autrement dit, aucune des deux caractéristiques précitées n'est réellement favorisée l'une par rapport à l'autre, en terme de dimension, contrairement, par exemple, aux seringues traditionnelles à aiguilles, pourvues d'un corps longiligne.

Des seringues sans aiguille à architecture compacte ont déjà été mises au point et ont fait l'objet de plusieurs brevets. On peut citer le brevet US 3,945,379 qui se rapporte à un dispositif d'injection sans aiguille présentant une géométrie compacte. En effet, ledit dispositif est constitué de deux segments linéaires, approximativement de même longueur, et faisant entre eux un angle droit. De cette façon, toute la partie motrice relative à la libération des gaz se retrouve désalignée de la partie incluant le système de poussée du principe actif liquide. Le déclenchement du dispositif est réalisé à l'aide d'une pression sur un bouton implanté dans la paroi latérale du segment comportant la réserve de gaz.

Les brevets US 5,383,851, US 5,399,163 et US 5,520,639 décrivent un dispositif d'injection hypodermique sans aiguille également d'architecture compacte. Ce dispositif se présente sous la forme de deux segments linéaires, l'un relatif à la partie motrice du dispositif, l'autre à l'expulsion du principe actif, ces deux segments étant parallèles et accolés l'un à l'autre tête-bêche. Le déclenchement du dispositif est provoqué par l'enfoncement d'un bouton placé à l'une des extrémités du segment contenant la réserve de gaz, ledit bouton ayant pour fonction de crever une réserve de gaz sous pression.

Enfin, le brevet EP 0853 952 concerne un système d'injection transdermique, de forme compacte et rechargeable. En effet ce système d'injection dispose d'un orifice dans lequel peut venir s'insérer un boîtier d'allumage autonome contenant le principe actif liquide. Après utilisation, ce boîtier est retiré du système puis remplacé par un autre boîtier prêt à l'usage. Le générateur fait intervenir une substance explosive qui est initiée électriquement au moyen d'une pile.

Les seringues sans aiguilles selon l'invention sont conçues pour répondre à un double objectif, qui est celui d'assurer une injection selon une direction perpendiculaire à la peau du patient, tout en garantissant une bonne sécurité d'utilisation par la limitation des possibilités de déclenchement à la seule configuration pour laquelle la partie qui est au contact de la peau et le système de déclenchement subissent simultanément une pression opposée de la part de l'utilisateur. Les seringues sans aiguille décrites dans l'Etat de la Technique sont de forme compacte, mais ne disposent d'aucun moyen spécifique en terme de forme, de géométrie ou de système de déclenchement leur permettant de remplir les deux objectifs précédents.

L'objet de la présente invention concerne une seringue sans aiguille comprenant un corps, un générateur de gaz, une chambre d'expansion desdits gaz, un réservoir contenant le principe actif et un système d'injection présentant au moins un canal d'injection caractérisée en ce que le corps a une architecture compacte induisant le désalignement du générateur de gaz et du réservoir contenant le principe actif, et le générateur de gaz est déclenché par un capot recouvrant ledit corps, ledit capot étant susceptible de coulisser le long dudit corps. Par ce biais, les seringues sans aiguille selon l'invention possèdent des dimensions ainsi qu'un mode de déclenchement qui les rendent utilisables d'une seule main, induisant un meilleur contrôle de la perpendicularité et permettant d'asseoir une plus grande stabilité de la seringue au moment de l'injection. Le mode de déclenchement qui se caractérise en particulier par un mouvement de translation du capot le long de l'axe de l'injection favorise également cette stabilité. De façon préférentielle, le générateur de gaz est un générateur de gaz pyrotechnique comprenant une charge pyrotechnique et un dispositif d'initiation.

Avantageusement, le système d'initiation comprend un dispositif de percussion et une amorce. Il est également possible d'utiliser un système d'initiation à base d'un cristal piézo-électrique ou d'un rugueux constitué par deux surfaces de frottement dont le déplacement crée une zone d'inflammation.

Préférentiellement, le dispositif de percussion inclut un ressort et un percuteur, ledit percuteur ayant la forme d'une pièce cylindrique creuse munie à l'une de ses extrémités d'une saillie et comportant sur sa surface latérale externe au moins deux aspérités présentant chacune un plan incliné incurvé, ledit percuteur enfermant ledit ressort.

De façon avantageuse, la pièce cylindrique creuse possède sur sa surface latérale externe au moins deux protubérances de forme sensiblement parallélépipédique, lesdites protubérances venant en appui contre au moins deux butées du corps pour empêcher le percuteur d'être translaté sous l'effet du ressort.

De façon préférentielle, le capot possède, d'une part, au moins deux ergots de blocage venant en appui contre les protubérances du percuteur pour empêcher ledit percuteur de subir une rotation autour de son axe et, d'autre part, au moins deux avancées se terminant chacune par un plan incliné incurvé, de sorte que le coulissement du capot le long du corps entraîne à la fois la translation des ergots pour libérer en rotation le percuteur et la translation desdites avancées qui viennent au contact des aspérités du percuteur au niveau de leurs plans inclinés respectifs qui s'emboîtent, provoquant la rotation dudit percuteur et le désalignement des protubérances avec les butées du corps. De façon plus concrète, le mode de fonctionnement de ce type de dispositif de percussion est le suivant : le percuteur qui enferme un ressort précontraint est bloqué en translation contre au moins une butée du corps. Le coulissement du capot induit une rotation du percuteur autour de son axe, avec un angle suffisamment important pour désaligner complètement ledit percuteur de la butée du corps. Le percuteur, qui est ainsi libéré en translation, est brutalement projeté vers l'amorce sous l'effet du ressort qui se détend. Un tel dispositif de percussion pourrait également être utilisé dans un dispositif d'initiation impliquant un cristal piézo-électrique.

De façon préférentielle, le capot dispose d'un moyen d'accrochage au corps lui permettant de rester dans une position d'enfoncement maximum. Selon un mode de réalisation préféré de l'invention, le moyen d'accrochage est constitué par une patte élastiquement déformable et dont l'extrémité est recroquevillée pour former un crochet. De cette manière, la position du capot enfoncé sur le corps apparaît instantanément comme un témoin d'utilisation.

Avantageusement, le générateur de gaz constitue un premier sous-ensemble linéaire du corps et le réservoir contenant le principe actif et le système d'injection forment un deuxième sous-ensemble linéaire dudit corps, les deux sous-ensembles faisant entre eux un angle inférieur ou égal à 90° et étant reliés entre eux par la chambre d'expansion. Cette disposition particulière des deux sous-ensembles est dictée par la géométrie compacte de la seringue.

Selon un mode de réalisation préféré de l'invention, les deux sous-ensembles ont des axes parallèles entre eux et sont reliés l'un à l'autre par la chambre d'expansion qui a un axe perpendiculaire aux axes desdits sous-ensembles.

Autrement dit, la seringue qui comprend successivement le dispositif de percussion, l'amorce, la charge pyrotechnique, la chambre d'expansion, le réservoir contenant le principe actif liquide et le système d'injection, a globalement une forme en 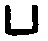 . Selon une variante d'exécution de l'invention, les deux sous-ensembles linaires peuvent se retrouver au contact l'un de l'autre.

Préférentiellement, le capot est apte à coulisser le long du corps selon un axe parallèle à ceux des deux sous-ensembles. Ce coulissement contribue à asseoir la stabilité de la seringue au moment de l'injection. De façon avantageuse, le rapport de la hauteur maximale de ladite seringue sur sa longueur maximale est compris entre 0,8 et 1,8, et préférentiellement entre 1,1 et 1,5. La hauteur de la seringue est la dimension de ladite seringue prise selon l'axe des deux sous-ensembles, et la longueur est la dimension prise selon un axe perpendiculaire aux axes des deux sous-ensembles et joignant ceux-ci. Avantageusement, la hauteur de la seringue est inférieure à 8 cm.

De façon préférentielle, un organe de compression est disposé entre le capot et le corps pour repousser ledit capot dudit corps. Cet organe, qui est préférentiellement constitué par un ressort, induit une résistance destinée à accroître l'effort pour enfoncer le capot le long du corps.

De façon avantageuse, le réservoir est constitué par un tube obturé par un bouchon-piston amont et un bouchon-piston aval entre lesquels est contenu le principe actif. Avantageusement, le tube est en verre.

De façon préférentielle, les deux bouchons-pistons sont réalisés en matériau déformable. Ils sont notamment obtenus par moulage d'élastomères compatibles avec le principe actif liquide sur une longue durée. Ces élastomères peuvent, par exemple, être des chlorobutyl ou des bromobutyl. Préférentiellement le système d'injection comporte une pièce terminale présentant un évidemment interne et au moins un canal d'injection périphérique, ledit évidemment étant destiné, durant l'injection, à recevoir le bouchon-piston aval tout en permettant le dégagement du canal d'injection.

En terme de fonctionnement, la colonne de liquide se déplace jusqu'à ce que le bouchon-piston aval vienne occuper l'évidement interne. Une fois bloqué dans ledit évidement, le bouchon-piston se déforme légèrement de façon à dégager l'entrée du canal d'injection périphérique et à permettre au principe actif d'être expulsé.

De façon avantageuse, le capot recouvre entièrement le corps et le système d'injection émerge dudit capot. Préférentiellement, le système d'injection et le capot coopèrent pour déclencher la seringue. En effet, la position relative du capot par rapport au corps de la seringue va conditionner le déclenchement de ladite seringue. Puisque, de façon préférentielle le système d'injection est solidaire du corps et vient au contact de la peau, c'est par son intermédiaire que le glissement du capot va s'effectuer. Il est donc nécessaire de disposer d'une surface d'appui pour déclencher la seringue.

Avantageusement le système d'injection se termine par une semelle de protection escamotable. Préférentiellement, ladite semelle a sensiblement les mêmes dimensions que le système d'injection et vient recouvrir étroitement ledit système.

Selon un mode de réalisation préféré de l'invention, le capot est prolongé par un bouchon recouvrant le système d'injection, ledit bouchon ayant sensiblement la même section que celle du capot qu'il prolonge. En réalité, le bouchon et la semelle escamotable ont la double fonction de sécuriser la seringue en empêchant tout coulissement du capot le long du corps et donc tout déclenchement intempestif de ladite seringue, et de protéger le système d'injection contre toute pollution parasite avant usage. De façon préférentielle, le bouchon
a) a un fond plan,
b) est relié au système d'injection par un système de baïonnettes,
c) obture le capot en étant à son contact et dans sa continuité.

Ces caractéristiques structurelles permettent à la seringue avant usage, d'être posée en équilibre stable sur une surface plane et de constituer un objet au contour lisse, d'encombrement réduit et dépourvu de toute aspérité inutile. Le système de baïonnettes permet de déverrouiller simplement la seringue, par une rotation de 90° du bouchon autour du système d'injection.

Les seringues sans aiguille selon l'invention possèdent un dispositif de déclenchement leur conférant une bonne fiabilité ainsi qu'un niveau de sécurité renforcé. En effet, un tel dispositif engendre un profil d'effort d'appui évolutif en fonction de l'enfoncement du capot, analogue à celui présenté à la figure 5 et distinguant trois phases. La première phase de ce profil correspond à une course réversible du capot sans déclenchement. Elle se traduit par un enfoncement du capot au cours duquel se produit la compression du ressort ou de la lame situé entre le capot et le corps. Au cours de cette phase, un relâchement de pression sur le capot entraîne un retour immédiat dudit capot à sa position initiale provoqué par la détente du ressort ou de la lame. La deuxième phase est relative à une course irréversible du capot assurant le déclenchement. Plus précisément, cette phase commence lorsque les plans inclinés hélicoïdaux complémentaires du capot et du percuteur viennent au contact les uns des autres, et se termine lorsque ledit percuteur, après avoir effectué une rotation suffisamment importante pour être désaligné des butées du corps, est libéré pour venir percuter l'amorce. Cette deuxième phase, outre l'effort à fournir pour continuer à contracter le ressort ou la lame situé entre le capot et le corps, nécessite un effort supplémentaire dû à la résistance offerte par le ressort précontraint logé dans le percuteur au moment de provoquer la rotation dudit percuteur. Enfin, la troisième phase se rapporte à une course libre du capot jusqu'au blocage, cette course correspondant à la poursuite de la compression du ressort ou de la lame situé entre le capot et le corps. L'effort à fournir durant cette phase est en continuité de celui à fournir lors de la première phase. Cette troisième phase s'achève lorsque le capot est arrivé en bout de course et qu'il est maintenu dans cette position par son organe de blocage. Globalement, un tel profil d'effort indique que la seringue selon l'invention ne peut pas être déclenchée par inadvertance ou par accident, et montre bien qu'il faut au contraire conduire une action volontaire pour la déclencher.

Les seringues sans aiguille selon l'invention ont l'avantage de se présenter sous la forme d'un objet compact, peu encombrant, dont la forme et les dimensions autorisent une utilisation aisée et naturelle avec une seule main. De plus, cette forme compacte, inhabituelle pour un dispositif d'injection, limite, voire supprime, l'appréhension que peut ressentir un patient dans la phase qui précède l'injection, augmentant ainsi l'efficacité du traitement en permettant qu'il soit fait jusqu'au bout. Enfin, les seringues sans aiguille, selon l'invention, présentent l'avantage de posséder un haut niveau de sécurité à partir de moyens simples et fonctionnels comme un bouchon autobloquant facilement déverrouillable, et particulièrement originaux comme le dispositif de percussion qui nécessite un effort progressif et particulier pour déclencher la seringue. Cette sécurité est accrue par le fait que le déclenchement de la seringue ne peut s'effectuer qu'à partir d'un positionnement bien précis de ladite seringue par rapport à la peau du patient à traiter, et du niveau d'effort d'appui calibré. La conjonction de cet effort et de ce positionnement perpendiculaire à la peau, permet de garantir un contact intime du système d'injection avec la peau et interdit tout risque de défaut d'appui lors de l'injection.

on donne ci-après la description détaillée d'un mode de réalisation préféré de l'invention en se référant aux figures 1 à 5.
La figure 1 est une vue en coupe longitudinale d'une seringue sans aiguille selon l'invention présentant un corps en forme de 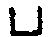 .
La figure 2 est une vue en perspective d'une seringue sans aiguille selon l'invention montrant la position du bouchon par rapport au capot.
Les figures 3a et 3b représentent chacune une vue en perspective du percuteur d'une seringue sans aiguille selon l'invention, les deux vues étant représentées chacune sous un angle particulier.
La figure 4 est une vue en coupe et en perspective du capot d'une seringue sans aiguille selon l'invention, le plan de coupe correspondant à son plan de symétrie.
La figure 5 est un graphique montrant l'effort à fournir pour enfoncer le capot en fonction du niveau d'enfoncement dudit capot.

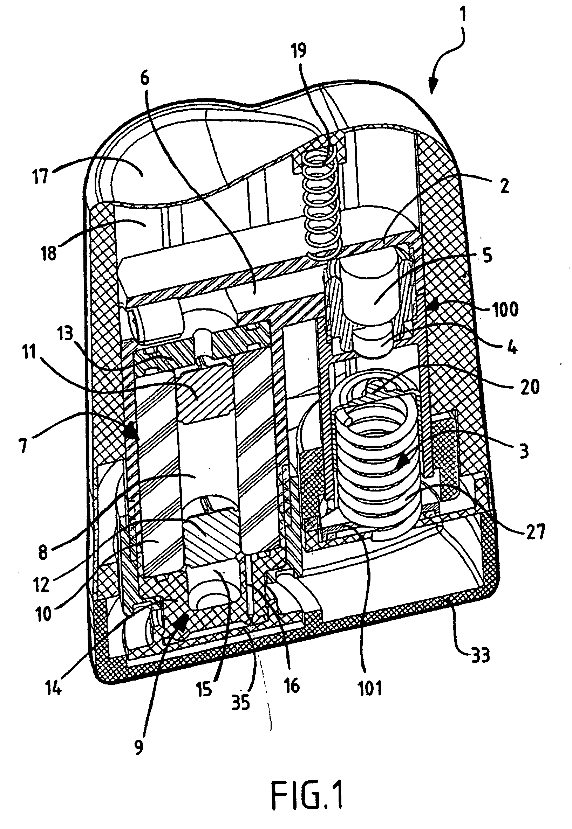

En se référant à la figure 1, une seringue 1 sans aiguille selon l'invention comporte un corps 2 en forme de 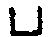 comprenant successivement un dispositif de percussion 3, une amorce 4, une charge pyrotechnique 5, ces trois éléments constituant un générateur de gaz 100, une chambre d'expansion 6, un réservoir 7 contenant le principe actif liquide 8 et un système d'injection 9. Le générateur de gaz 100 constitue un premier sous-ensemble linéaire du corps 2, et, le réservoir 7 contenant le principe actif 8 et le système d'injection 9 forment un deuxième sous-ensemble linéaire dudit corps 2, ces deux sous-ensembles ayant des axes parallèles entre eux et étant reliés l'un à l'autre par la chambre d'expansion 6 qui a un axe perpendiculaire aux axes desdits sous-ensembles. Le réservoir 7 est constitué par un tube 10 en verre obturé par un bouchon-piston amont 11 et un bouchon-piston aval 12 entre lesquels est contenu le principe actif liquide 8, lesdits bouchons-pistons étant réalisés en matériau déformable à base d'élastomère. Le réservoir 7 est inséré dans le corps 2 de façon à ce que sa paroi latérale externe soit au contact dudit corps 2 et est fixé longitudinalement, d'une part, à sa partie amont par une pièce cylindrique 13 munie d'une ouverture centrale permettant de mettre en communication le bouchon-piston amont 11 avec la chambre d'expansion 6, ladite pièce 13 servant de tampon entre l'une des extrémités dudit réservoir 7 et le corps 2 et, d'autre part, à sa partie aval par une pièce cylindrique creuse 14, obturée à l'une de ses extrémités et présentant une collerette à son autre extrémité ouverte. Cette pièce cylindrique creuse 14, qui constitue la pièce principale du système d'injection 9, est fixée autour du réservoir 7 comme un capuchon, ledit réservoir 7 venant en butée contre la collerette. Ainsi positionnée, ladite pièce creuse 14 ménage un espace libre assimilable à un évidement interne 15, ledit espace ayant approximativement les dimensions du bouchon-piston aval 12 et étant situé juste derrière lui, dans sa continuité. La pièce cylindrique creuse 14 présente dans son épaisseur, trois canaux d'injection périphériques 16 parallèles à son axe et qui débouchent, d'une part, dans la face obturée de ladite pièce 14, et, d'autre part, dans la partie supérieure de l'évidement 15 située près du bouchon-piston aval 12. Cette pièce creuse 14 qui constitue l'essentiel du système d'injection 9 évite à l'extrémité du tube 10 en verre d'être directement au contact du corps 2.

Un capot 17, muni d'une ouverture, enveloppe intégralement le corps 2. Il se présente sous la forme de deux demi-coquilles identiques, accolées l'une à l'autre, et il se positionne autour dudit corps 2 en étant à son contact au niveau de la paroi latérale externe de ses deux sous-ensembles et en ménageant un espace 18 entre le segment du corps 2 matérialisé par la chambre d'expansion 6 et la paroi interne du sommet du capot 17 qui est la partie fermée dudit capot 17 située à l'opposé de son ouverture. Un ressort 19 à spirales, dont l'axe est parallèle à celui des deux sous-ensembles, est en appui à ses deux extrémités, d'une part, contre la paroi interne du sommet du capot 17 et, d'autre part, contre le corps 2 au niveau de la paroi externe de la chambre d'expansion 6. Ledit ressort 19 est stabilisé latéralement par deux ergots opposés et alignés, l'un émergeant de la face interne du sommet du capot 17, l'autre émergeant de la face externe du corps 2 au niveau de la chambre d'expansion 6. Lorsque le capot 17 est ainsi positionné autour du corps 2, seule émerge de son ouverture une partie de la pièce cylindrique creuse 14 du système d'injection 9.

Le générateur de gaz 100 comprend trois parties distinctes et alignées :
- un système de percussion 3 mettant en oeuvre un percuteur 20 et un ressort 27 précontraint logé à l'intérieur dudit percuteur 20
- une amorce 4 classique de type amorce de cartouche de chasse,
- une charge pyrotechnique 5 constituée d'une poudre apte à émettre une grande quantité de gaz comme, par exemple, une poudre simple base à la nitrocellulose.

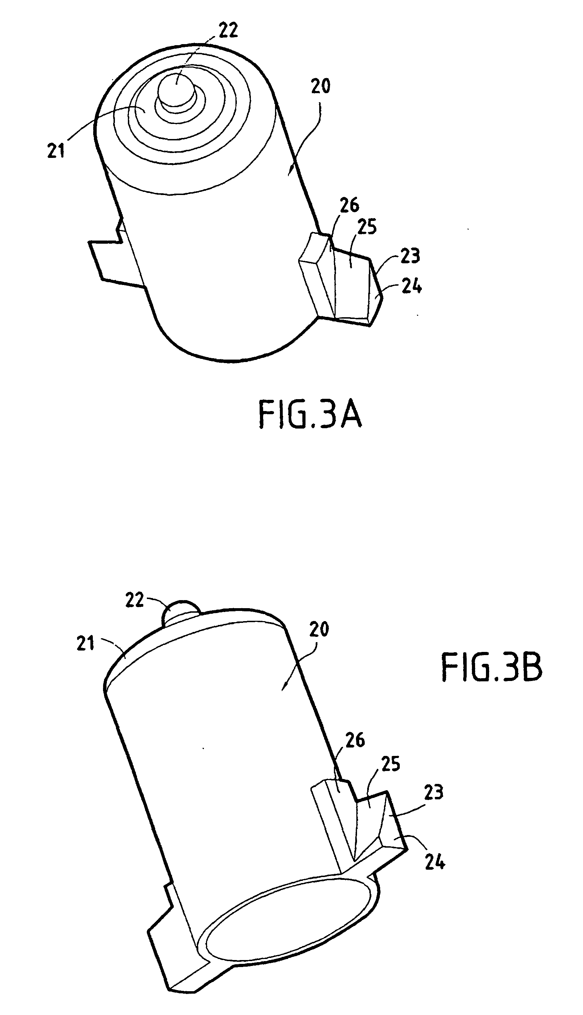

En se référant aux figures 3a et 3b, le percuteur 20 est constitué d'une pièce cylindrique creuse, ouverte à l'une de ses extrémités, et fermée à son autre extrémité par un couvercle 21 de forme concave par rapport au corps de ladite pièce cylindrique creuse, le sommet dudit couvercle 21 se terminant par une saillie 22 arrondie. Les trois éléments constitutifs du générateur de gaz 100 sont positionnés de sorte qu'ils se retrouvent alignés en ayant leur axe de symétrie confondu, le système de percussion 3 étant la partie la plus en amont dudit générateur 100, et la saillie 22 arrondie étant la partie du percuteur 20 la plus proche de l'amorce 4.

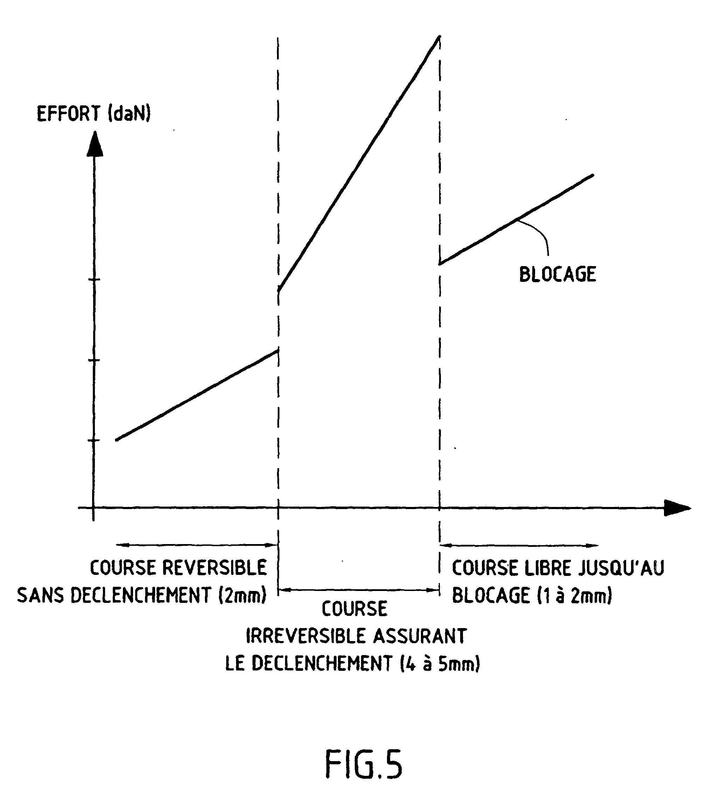

Le percuteur 20 présente sur sa surface latérale externe, deux aspérités 23 diamétralement opposées et situées dans la zone la plus proche de son ouverture. Chacune de ses aspérités 23 présente un sommet plan 24 ayant sensiblement la forme d'un triangle rectangle dont l'un des côtés est parallèle à l'axe du percuteur 20 et l'autre est perpendiculaire audit axe. Lesdites aspérités 23 comportent un plan incliné incurvé 25 assimilable à une portion hélicoïdale, l'inclinaison dudit plan 25 étant matérialisée par le segment joignant les deux côtés du sommet triangulaire 24, autrement dit par l'hypotenuse dudit triangle. La configuration d'une aspérité 23 se déduit de celle de l'autre aspérité par une rotation de 180° autour de l'axe du percuteur 20. Le percuteur 20 possède également sur sa surface latérale externe deux protubérances 26 de forme sensiblement parallélépipédique, présentant un bord parallèle à l'axe dudit percuteur 20 et un autre perpendiculaire audit axe. Chaque protubérance 26 est située entre chaque aspérité 23 et la paroi latérale externe du percuteur 20. Le ressort 27 qui est logé dans le percuteur 20 est précontraint et prend appui sur une plaque 101 de fermeture de la base du générateur 100, ladite plaque 101 étant située à l'opposé de la saillie 22 par rapport au percuteur 20.
En se référant à la figure 1, le percuteur 20 est positionné dans le générateur 100 de manière à ce qu'il vienne en appui contre deux butées dudit générateur 100, au niveau de ses deux protubérances 26 et, plus précisément, au niveau du bord supérieur de chacune des deux protubérances 26, ledit bord étant perpendiculaire à l'axe du percuteur 20. En se référant à la figure 4, le capot 17 présente sur sa face interne deux ergots 29 plans, placés chacun sur une demi-coquille , en position symétrique l'un par rapport à l'autre, lesdits ergots 29 venant en appui contre les protubérances 26 du percuteur 20 et, plus précisément, contre l'un des deux bords des protubérances 26 qui est parallèle à l'axe dudit percuteur 20.

Le capot 17 comporte également deux avancées 30 se terminant chacune par un plan incliné 31 incurvé assimilable à une portion hélicoïdale. Lesdites avancées 30 sont placées au droit des aspérités 23 du percuteur 20 de sorte que les plans inclinés 25 des aspérités 23 du percuteur 20 se retrouvent en face des plans inclinés 31 des avancées 30 du capot 17 et dans des positions complémentaires. Autrement dit, si les plans inclinés 31 terminant les avancées 30 du capot 17 devaient être mis au contact des plans inclinés 25 des aspérités 23 du percuteur 20 par une simple translation, ils s'emboîteraient parfaitement. Le capot 17 dispose d'une patte 32 élastiquement déformable et dont l'extrémité est recroquevillée pour former un crochet. Il s'agit d'un dispositif d'accrochage permettant au capot 17, une fois qu'il a coulissé le long du corps 2 de conserver sa position finale correspondant à un enfoncement maximum.

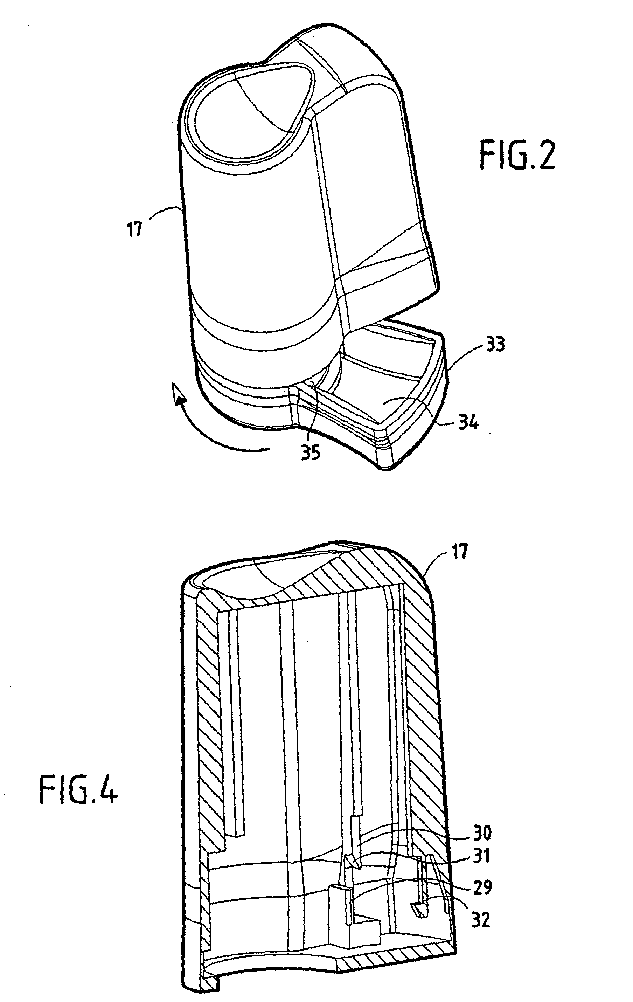

En se référant à la figure 2, une seringue sans aiguille 1 selon l'invention dispose d'un bouchon 33 de fermeture ayant un fond plan 34 et sensiblement la même section que celle constituée par l'ouverture du capot 17. Ainsi, munie de son bouchon 33, la seringue 1 se présente sous la forme d'un objet lisse et homogène n'exhibant ni aspérité, ni cassures de relief. Le bouchon 33 est fixé à la seringue 1 au niveau de la partie de la pièce cylindrique creuse 14 du système d'injection 9 qui émerge du capot 17. Plus précisément, le bouchon 33 possède sur son fond 34 un petit capuchon 35 qui vient se fixer autour de l'extrémité de ladite pièce creuse 14 qui émerge du capot 17, par un système de baïonnettes. Une simple rotation de 90° permet de retirer ledit bouchon 33 qui protège ledit système 9 grâce à son capuchon 35 et empêche tout coulissement du capot 17 le long du corps 2.

Le mode de fonctionnement d'une seringue 1 sans aiguille selon l'invention s'effectue comme suit :
L'utilisateur déverrouille la seringue 1 en ôtant le bouchon 33 par une rotation de 90° comme cela est indiqué par la flèche sur la figure 2. Il applique le système d'injection 9 contre la peau du patient à traiter et, par pression d'un doigt, il enfonce le capot 17 qui coulisse le long du corps 2 jusqu'à ce que les plans inclinés 31 incurvés des avancées 30 dudit capot 17 viennent au contact des plans inclinés 25 incurvés du percuteur 20 qui leur font face. Durant cette première phase, il a fallu fournir un effort modéré pour juste comprimer le ressort 19 compris entre le corps 2 et le capot 17. En continuant la pression sur le capot 17, les ergots 29 qui étaient au contact des protubérances 26 du percuteur 20 se translatent jusqu'à ne plus être au contact desdites protubérances 26, provoquant le déblocage du percuteur 20 en rotation. simultanément, les avancées 30 appuient sur les aspérités 23 du percuteur 20 provoquant, grâce aux plans inclinés incurvés 25, 31 qui se complètent, la rotation dudit percuteur 20. L'amplitude de cette rotation est telle qu'elle provoque le désalignement des butées du générateur 100 avec les protubérances 26 du percuteur 20 qui, ne se trouvant plus bloqué, est accéléré pour impacter avec sa saillie 22 l'amorce 4 sous l'effet de la détente du ressort 27 qu'il renferme. Durant cette phase, les efforts à fournir ont été accrus par le fait qu'il a fallu vaincre, pendant la rotation du percuteur 20, la résistance offerte par le ressort 27 précontraint logé dans ledit percuteur 20. L'initiation de l'amorce 4 entraîne la mise à feu de la charge pyrotechnique 5 qui, par combustion, se décompose en fournissant des gaz. Les gaz envahissent la chambre d'expansion 6 et, lorsque la pression est suffisante, exercent une poussée sur la colonne de liquide constituée par les deux bouchons-pistons 11, 12 et le principe actif liquide 8. Le bouchon-piston aval 12 vient occuper complètement l'évidement interne 15 permettant au principe actif liquide 8 de déboucher dans les canaux d'injection périphériques 16. La poussée continuant de s'exercer sur le bouchon-piston amont 11, le principe actif liquide 8 est alors expulsé par les canaux 16 jusqu'à ce que le bouchon-piston amont 11 vienne au contact du bouchon-piston aval 12.
Juste après que le percuteur 20 impacte l'amorce 4 pour déclencher la seringue 1, le capot 17 continue d'être enfoncé sans effort marqué, dans la continuité du mouvement, et finit par arrêter de se déplacer lorsque le ressort 19 situé entre ledit capot 17 et le corps 2 est comprimé au maximum. Durant le coulissement du capot 17 le long du corps 2, la patte 32 a glissé le long du corps 2 en se déformant élastiquement pour respecter le relief dudit corps 2 et a fini par s'accrocher au rebord du corps 2 avec son extrémité repliée. Après usage, le capot 17 reste donc en position enfoncée.

## Revendications

1. Seringue sans aiguille (1) comprenant un corps (2), un générateur de gaz (100), une chambre d'expansion (6) desdits gaz, un réservoir (7) contenant le principe actif (8) et un système d'injection (9) présentant au moins un canal d'injection (16), ledit corps (2) ayant une architecture compacte induisant le désalignement du générateur de gaz (100) et du réservoir (7) contenant le principe actif (8), **caractérisée en ce que** le générateur de gaz (100) est déclenché par un capot (17) recouvrant ledit corps (2), ledit capot (17) étant susceptible de coulisser le long dudit corps (2) pour engendrer le déclenchement lorsque la seringue est déverouillée.

2. Seringue sans aiguille selon la revendication 1, **caractérisée en ce que** le générateur de gaz (100) est un générateur de gaz pyrotechnique comprenant une charge pyrotechnique (5) et un dispositif d'initiation.

3. Seringue sans aiguille selon la revendication 1, **caractérisée en ce que**, d'une part, le générateur de gaz (100) constitue un premier sous-ensemble linéaire du corps (2) et, d'autre part, le réservoir (7) contenant le principe actif (8) et le système d'injection (9) forment un deuxième sous-ensemble linéaire dudit corps (2), les deux sous-ensembles faisant entre eux un angle inférieur ou égal à 90° et étant reliés entre eux par la chambre d'expansion (6).

4. Seringue sans aiguille selon la revendication 3, **caractérisée en ce que** les deux sous-ensembles ont des axes parallèles entre eux et sont reliés l'un à l'autre par la chambre d'expansion (6) qui a un axe perpendiculaire aux axes desdits sous-ensembles.

5. Seringue sans aiguille selon la revendication 4, **caractérisée en ce que** le capot (17) est apte à coulisser le long du corps (2) selon un axe parallèle à ceux des deux sous-ensembles.

6. Seringue sans aiguille selon l'une quelconque des revendications 1 ou 5, **caractérisée en ce que** le rapport de la hauteur maximale de ladite seringue (1) sur sa longueur maximale est compris entre 0,8 et 1,8.

7. Seringue sans aiguille selon la revendication 1, **caractérisée en ce qu'**un organe de compression (19) est disposé entre le capot (17) et le corps (2) de façon à repousser ledit capot (17) dudit corps (2).

8. Seringue sans aiguille selon la revendication 1 **caractérisée en ce que** le réservoir (7) est constitué par un tube (10) obturé par un bouchon-piston amont (11) et un bouchon-piston aval (12) entre lesquels est contenu le principe actif (8).

9. Seringue sans aiguille selon la revendication 1, **caractérisée en ce que** le capot (17) recouvre entièrement le corps (2) et le système d'injection (9) émerge dudit capot (17).

10. Seringue sans aiguille selon l'une quelconque des revendications 1 à 9, **caractérisée en ce que** le système d'injection (9) et le capot (17) coopèrent pour déclencher ladite seringue (1).

11. Seringue sans aiguille selon l'une quelconque des revendications 1 ou 9, **caractérisée en ce que** le système d'injection (9) se termine par une semelle de protection escamotable.

12. Seringue sans aiguille selon l'une quelconque des revendications 1 ou 9 **caractérisée en ce que** le capot (17) est prolongé par un bouchon (33) recouvrant le système d'injection (9), ledit bouchon (33) ayant sensiblement la même section que celle du capot (17) qu'il prolonge.

13. Seringue sans aiguille selon la revendication 12 **caractérisée en ce que** le bouchon (33)
a) a un fond plan (34),
b) est relié au système d'injection (9) par un système de baïonnettes,
c) obture le capot (17) en étant à son contact et dans sa continuité.

14. Seringue sans aiguille selon la revendication 2 **caractérisée en ce que** le dispositif d'initiation comprend une amorce (4) et un dispositif de percussion (3) qui inclut un ressort (27) et un percuteur (20), ledit percuteur (20) ayant la forme d'une pièce cylindrique creuse munie à l'une de ses extrémités d'une saillie (22) et comportant sur sa surface latérale externe au moins deux aspérités (23) présentant chacune un plan incliné incurvé (25), ledit percuteur (20) enfermant ledit ressort (27).

15. Seringue sans aiguille selon la revendication 14 **caractérisée en ce que** la pièce cylindrique creuse possède sur sa surface latérale externe au moins deux protubérances (26) de forme sensiblement parallélépipédique, lesdites protubérances (26) venant en appui contre au moins deux butées du corps (2) pour empêcher le percuteur (20) d'être translaté sous l'effet du ressort (27).

16. Seringue sans aiguille selon la revendication 15 **caractérisée en ce que** le capot (17) possède, d'une part, au moins deux ergots (29) de blocage venant en appui contre les protubérances (26) du percuteur (20) pour empêcher ledit percuteur (20) de subir une rotation autour de son axe et, d'autre part, au moins deux avancées (30) se terminant chacune par un plan incliné incurvé (31), de sorte que le coulissement du capot (17) le long du corps (2) entraîne à la fois la translation des ergots (29) pour libérer en rotation le percuteur (20) et la translation desdites avancées (30) qui viennent au contact des aspérités (23) du percuteur (20) au niveau de leurs plans inclinés respectifs (25, 31) qui s'emboîtent, provoquant la rotation dudit percuteur (20) et le désalignement des protubérances (26) avec les butées du corps (2).

17. Seringue sans aiguille selon la revendication 1 **caractérisée en ce que** le capot (17) dispose d'un moyen d'accrochage (32) au corps (2) lui permettant de rester dans une position d'enfoncement maximum.

## Claims

1. Needleless syringe (1) comprising a body (2), a gas generator (100) an expansion chamber (6) for the said gases, a reservoir (7) containing the active principle (8) and an injection system (9) having at least one injection channel (16) the said body (2) having a compact architecture inducing a misalignment of the gas generator (100) and the reservoir (7) containing the active principle (8) **characterised in that** the gas generator (100) is triggered by a cap (17) covering the said body (2), the said cap (17) being capable of sliding along the said body (2) so as to bring about triggering when the syringe is unlocked.

2. Needleless syringe according to claim 1, **characterised in that** the gas generator (100) is a pyrotechnic gas generator comprising a pyrotechnic charge (5) and an initiation device.

3. Needleless syringe according to claim 1, **characterised in that**, on the one hand, the gas generator (100) constitutes a first linear sub-assembly of the body (2) and, on the other hand, the reservoir (7) containing the active principle (8) and the injection system (9) form a second linear sub-assembly of the said body (2), the two sub-assemblies making between them an angle of less than or equal to 90° and being connected together by the expansion chamber (6).

4. Needleless syringe according to claim 3, **characterised in that** the two sub-assemblies have axes that are parallel to each other and are connected to each other by the expansion chamber (6) which has an axis perpendicular to the axes of the said sub-assemblies.

5. Needleless syringe according to claim 4, **characterised in that** the cap (17) is capable of sliding along the body (2) along an axis parallel to those of the two sub-assemblies.

6. Needleless syringe according to either of claims 1 or 5, **characterised in that** the ratio of the maximum height of the said syringe (1) to its maximum length lies between 0.8 and 1.8.

7. Needleless syringe according to claim 1, **characterised in that** a compression device (19) is positioned between the cap (17) and the body (2) so as to push back the said cap (17) of the said body (2).

8. Needleless syringe according to claim 1, **characterised in that** the reservoir (7) consists of a tube (10) closed by an upstream stopper-piston (11) and a downstream stopper-piston (12) between which the active principle (8) is contained.

9. Needleless syringe according to claim 1, **characterised in that** the cap (17) entirely covers the body (2) and the injection system (9) emerges from the said cap (17).

10. Needleless syringe according to any one of claims 1 to 9, **characterised in that** the injection system (9) and the cap (17) cooperate in order to trigger the said syringe (1).

11. Needleless syringe according to either of claims 1 or 9, **characterised in that** the injection system (9) ends in a retractable protective base.

12. Needleless syringe according to either of claims 1 or 9, **characterised in that** the cap (17) is extended by a stopper (33) covering the injection system (9), the said stopper (33) having substantially the same cross section as that of the cap (17) that it extends.

13. Needleless syringe according to claim 12, **characterised in that** the stopper (33)
a) has a flat bottom (34),
b) is connected to the injection system (9) by a system of bayonets
c) closes the cap (17) while being in contact with it and being in an extension of it.

14. Needleless syringe according to claim 2, **characterised**
**in that** the initiation device comprises a detonator (4) and a percussion device (3) which includes a spring (27) and a striker (20), the said striker (20) having the shape of a hollow cylindrical piece provided at one of its ends with a projection (22) and including on its external lateral surface at least two asperities (23), each having an inwardly curved incline plane (25), the said striker (20) enclosing the said spring (27).

15. Needleless syringe according to claim 14, **characterised in that** the hollow cylindrical piece possesses on its external lateral surface at least two protuberances (26) with a substantially parallelepiped form, the said protuberances (26) resting against at least two stops of the body (2) so as to prevent the striker (20) from being moved in translation under the effect of the spring (27).

16. Needleless syringe according to claim 15, **characterised in that** the cap (17) possesses, on the one hand, at least two blocking tabs (29) resting against the protuberances (26) of the striker (20) so as to prevent the said striker (20) from undergoing a rotation about its axis and, on the other hand, at least two leading portions (30) each ending in an inwardly curving inclined plane (31), so that sliding the cap (17) along the body (2) brings about at the same time the movement in translation of the tabs (29) so as to free the striker (20) in rotation and the movement in translation of the said leading portions (30) which come into contact with the asperities (23) of the striker (20) in the region of their respective inclined planes (25, 31) which fit into each other, bring about the rotation of the said striker (20) and the misalignment of the protuberances (26) with the stops of the body (2).

17. Needleless syringe according to claim 1, **characterised in that** the cap (17) has available a means of engagement (32) with the body (2) enabling it to remain in a position of maximum depression.

## Patentansprüche

1. Nadellose Spritze (1) mit einem Körper (2), einem Gasgenerator (100), einer Ausdehnungskammer (6) für die Gase, einem den aktiven Wirkstoff (8) enthaltenden Behälter (7) und einen Injektionssystem (9), das mindestens einen Injektionskanal (16) aufweist, wobei der Körper (2) eine kompakte Struktur hat, die eine nicht fluchtende Anordnung des Gasgenerators (100) und des den aktiven Wirkstoff (8) enthaltenden Behälters (7) bewirkt, **dadurch gekennzeichnet, dass** der Gasgenerator (100) von einer den Körper (2) bedeckenden Kappe (17) ausgelöst wird, wobei die Kappe (17) in der Lage ist, entlang des Körpers (2) zu gleiten, um das Auslösen zu erzeugen, wenn die Spritze entriegelt wird.

2. Nadellose Spritze nach Anspruch 1, **dadurch gekennzeichnet, dass** der Gasgenerator (100) ein pyrotechnischer Gasgenerator ist, der eine pyrotechnische Ladung (5) und eine Zündvorrichtung aufweist.

3. Nadellose Spritze nach Anspruch 1, **dadurch gekennzeichnet, dass** der Gasgenerator (100) einerseits eine erste lineare Untereinheit des Körpers (2) und der den aktiven Wirkstoff (8) enthaltende Behälter (7) und das Injektionssystem (9) andererseits eine zweite lineare Untereinheit des Körpers (2) bilden, wobei die beiden Untereinheiten zueinander einen Winkel von weniger als oder gleich 90° bilden und über die Ausdehnungskammer (6) miteinander verbunden sind.

4. Nadellose Spritze nach Anspruch 3, **dadurch gekennzeichnet, dass** die beiden Untereinheiten zueinander parallele Achsen aufweisen und über die Ausdehnungskammer (6) miteinander verbunden sind, die eine Achse senkrecht zu den Achsen der Untereinheiten aufweist.

5. Nadellose Spritze nach Anspruch 4, **dadurch gekennzeichnet, dass** die Kappe (17) entlang des Körpers (2) gemäß einer Achse parallel zu denjenigen der beiden Untereinheiten gleiten kann.

6. Nadellose Spritze nach einem der Ansprüche 1 oder 5, **dadurch gekennzeichnet, dass** das Verhältnis der maximalen Höhe der Spritze (1) zu ihrer maximalen Länge zwischen 0,8 und 1,8 liegt.

7. Nadellose Spritze nach Anspruch 1, **dadurch gekennzeichnet, dass** ein Druckorgan (19) zwischen der Kappe (17) und dem Körper (2) angeordnet ist, um die Kappe (17) vom Körper (2) wegzudrücken.

8. Nadellose Spritze nach Anspruch 1, **dadurch gekennzeichnet, dass** der Behälter (7) aus einem Rohr (10) besteht, das von einem stromaufwärts liegenden Stopfen-Kolben (11) und einem stromabwärts liegenden Stopfen-Kolben (12) verschlossen wird, zwischen denen der aktive Wirkstoff (8) enthalten ist.

9. Nadellose Spritze nach Anspruch 1, **dadurch gekennzeichnet, dass** die Kappe (17) den Körper (2) vollständig bedeckt und das Injektionssystem (9) aus der Kappe (17) vorsteht.

10. Nadellose Spritze nach einem der Ansprüche 1 bis 9, **dadurch gekennzeichnet, dass** das Injektionssystem (9) und die Kappe (17) zusammenwirken, um die Spritze (1) auszulösen.

11. Nadellose Spritze nach einem der Ansprüche 1 oder 9, **dadurch gekennzeichnet, dass** das Injektionssystem (9) in einer einziehbaren Schutzplatte endet.

12. Nadellose Spritze nach einem der Ansprüche 1 oder 9, **dadurch gekennzeichnet, dass** die Kappe (17) von einem Stopfen (33) verlängert wird, der das Injektionssystem (9) bedeckt, wobei der Stopfen (33) im wesentlichen den gleichen Querschnitt wie die Kappe (17) hat, die er verlängert.

13. Nadellose Spritze nach Anspruch 12, **dadurch gekennzeichnet, dass** der Stopfen (33)
a) einen ebenen Boden (34) aufweist,
b) mit dem Injektionssystem (9) über ein Bajonett-System verbunden ist,
c) die Kappe (17) verschließt, indem er mit ihr in Kontakt steht und sie verlängert.

14. Nadellose Spritze nach Anspruch 2, **dadurch gekennzeichnet, dass** die Zündvorrichtung einen Zünder (4) und eine Schlagvorrichtung (3) aufweist, die eine Feder (27) und einen Schlagbolzen (20) enthält, wobei der Schlagbolzen (20) die Form eines hohlen zylindrischen Bauteils hat, das an einem seiner Enden mit einem Vorsprung (22) versehen ist und auf seiner seitlichen Außenfläche mindestens zwei Unebenheiten (23) aufweist, die je eine gekrümmte geneigte Ebene (25) haben, wobei der Schlagbolzen (20) die Feder (27) umschließt.

15. Nadellose Spritze nach Anspruch 14, **dadurch gekennzeichnet, dass** das hohle zylindrische Bauteil auf seiner seitlichen Außenfläche mindestens zwei Ausstülpungen (26) von im wesentlichen parallelepipedischer Form aufweist, die gegen mindestens zwei Anschläge des Körpers (2) anliegen, um eine Translationsverschiebung des Schlagbolzens (20) unter der Wirkung der Feder (27) zu verhindern.

16. Nadellose Spritze nach Anspruch 15, **dadurch gekennzeichnet, dass** die Kappe (17) einerseits mindestens zwei Blockiernocken (29), die gegen die Ausstülpungen (26) des Schlagbolzens (20) in Anlage kommen, um den Schlagbolzen (20) an einer Drehung um seine Achse zu hindern, und andererseits mindestens zwei Auflieger (30) aufweist, die je in einer geneigten gekrümmten Ebene (31) enden, so dass das Gleiten der Kappe (17) entlang des Körpers (2) sowohl die Translationsbewegung der Nocken (29), um den Schlagbolzen (20) in Drehung freizusetzen, als auch die Translationsbewegung der Auflieger (30) bewirkt, die mit den Unebenheiten (23) des Schlagbolzens (20) in Höhe ihrer geneigten Ebenen (25 bzw. 31) in Kontakt gelangen, die sich ineinander fügen, wodurch die Drehung des Schlagbolzens (20) und die nicht mehr fluchtende Anordnung der Ausstülpungen (26) zu den Anschlägen des Körpers (2) bewirkt werden.

17. Nadellose Spritze nach Anspruch 1, **dadurch gekennzeichnet, dass** die Kappe (17) über ein Befestigungsmittel (32) am Körper (2) verfügt, das es ihr erlaubt, in einer Stellung des maximalen Eindrückens zu bleiben.
